# EUROPEAN PATENT SPECIFICATION

(11) **EP 3 076 864 B1**
(45) Date of publication and mention of the grant of the patent: **10.03.2021**
(21) Application number: 14867454.2
(22) Date of filing: 05.12.2014
(51) Int. Cl.: A61B 8/06, A61M 25/02, A61M 25/00, A61M 25/01, A61B 8/12, A61B 5/00, A61B 5/0215

(54) **DEVICE FOR ASSESSING INTRAVASCULAR PRESSURE**
VORRICHTUNG ZUR BEWERTUNG VON INTRAVASKULÄREM DRUCK
DISPOSITIF DE MESURE DE LA PRESSION INTRAVASCULAIRE

(30) Priority: 06.12.2013 US 201361913065 P; 06.12.2013 US 201361913160 P
(43) Date of publication of application: 12.10.2016
(73) Proprietor: Philips Image Guided Therapy Corporation, San Diego CA 92130 (US)
(72) Inventor: STIGALL, Jeremy, Carlsbad, California 92009 (US)
(74) Representative: Philips Intellectual Property & Standards
(86) International application number: PCT/US2014/068874
(87) International publication number: WO 2015/085220

(56) References cited:
- WO-A1-2011/069505
- US-A- 4 497 324
- US-A1- 2002 095 141
- US-A1- 2008 140 180
- US-A1- 2008 319 278
- US-A1- 2010 241 069
- US-A1- 2011 092 955
- US-A1- 2012 220 883
- US-A1- 2013 190 803
- US-A1- 2013 289 369
- US-A1- 2013 303 914

## Description

### TECHNICAL FIELD

Embodiments of the present disclosure relate generally to the field of medical devices and, more particularly, to a device, system, and method for assessing pressure within vessels. In particular, the present disclosure relates to the assessment of the severity of a blockage or other restriction to the flow of fluid through a vessel. Aspects of the present disclosure are particularly suited for evaluation of biological vessels in some instances. For example, some particular embodiments of the present disclosure are specifically configured for the evaluation of a stenosis of a human blood vessel.

### BACKGROUND

Heart disease is a serious health condition affecting millions of people worldwide. One major cause of heart disease is the presence of blockages or lesions within the blood vessels that reduce blood flow through the vessels. Traditionally, surgeons have relied on X-ray fluoroscopic (planar) images to show the external shape or silhouette of the blood vessels to guide treatment. Unfortunately, using only X-ray fluoroscopic images lends a great deal of uncertainty about the exact extent and orientation of the lesion responsible for the occlusion, making it difficult to find the exact location of the stenosis for treatment. In addition, X-ray fluoroscopy is an inadequate reassessment tool to evaluate the vessel after surgical treatment.

A currently accepted technique for assessing the severity of a stenosis in a blood vessel, including ischemia-causing lesions, is fractional flow reserve (FFR). FFR is defined as the ratio of the maximal blood flow in a stenotic artery, taken distal to the lesion, to normal maximal flow. Accordingly, to calculate the FFR for a given stenosis, two blood pressure measurements are taken: one measurement distal or downstream to the stenosis and one measurement proximal or upstream to the stenosis. FFR is a calculation of the ratio of the distal pressure measurement relative to the proximal pressure measurement. FFR provides an index of stenosis severity that allows determination as to whether the blockage limits blood flow within the vessel to an extent that treatment is required. The more restrictive the stenosis, the greater the pressure drop across the stenosis, and the lower the resulting FFR. FFR measurements can be used as a decision point for guiding treatment decisions. The normal value of FFR in a healthy vessel is 1.00, while values less than about 0.80 are generally deemed significant and require treatment. Common treatment options include angioplasty, atherectomy, and stenting.

One method of measuring the pressure gradient across a lesion is to use a pressure sensing guidewire that has a pressure sensor embedded within the guidewire itself. A user may initially position the pressure sensor of the guidewire distal to the lesion and measure the distal pressure before drawing the guidewire backwards to reposition the sensor proximal to the lesion to measure the proximal pressure. This method has the disadvantages of inaccurate pressure readings due to drift and increased susceptibility to thermal variations, difficulty maneuvering a guidewire through blockages, high manufacturing costs, and time-consuming repositioning steps (especially in situations involving multiple lesions). Further, pressure-sensing guidewires often suffer from reduced precision and accuracy in making intravascular pressure measurements when compared to larger pressure-sensing devices, such as aortic pressure-sensing catheters.

Another method of measuring the pressure gradient across a lesion is to use a small catheter connected to a blood pressure sensor, which is often contained in a sensor housing associated with the catheter. However, this method can introduce error into the FFR measurement because as the catheter crosses the lesion, the catheter and the sensor housing themselves create additional blockage to blood flow across the lesion and contributes to a lower distal blood pressure than what would be caused by the lesion alone, which may exaggerate the measured pressure gradient across the lesion.

While the existing treatments have been generally adequate for their intended purposes, they have not been entirely satisfactory in all respects. The devices, systems, and associated methods of the present disclosure overcome one or more of the shortcomings of the prior art

US 2013/0190803 relates to the field of vascular filters for capturing embolic material in the blood flow.

### SUMMARY

The invention is as specified in the claims.

In one exemplary embodiment, the present disclosure describes an apparatus for a micro-catheter having a pressure sensor and a centering assembly to generally center the catheter within the vessel of interest. In still a further aspect, the sensing micro-catheter has in internal lumen with a diameter large enough to receive a 0.356 mm (0.014 inch) diameter guidewire and an outside diameter of 0.889 mm (0.035 inch) or less.

In further embodiments, the present disclosure relates generally to a device, systems, and methods of using a pressure-sensing catheter for the assessment of intravascular pressure, including, by way of non-limiting example, the calculation of a FFR value. In some instances, embodiments of the present disclosure are configured to measure the pressure proximal to and distal to a stenotic lesion within a blood vessel. Embodiments of the present disclosure include a pressure sensor embedded in the wall of the catheter or have a movable sleeve capable of smoothing the outer diameter of the sensing catheter. In some embodiments, the pressure-sensing catheter disclosed herein is configured as a monorail or rapid exchange catheter where the guidewire exits the catheter body adjacent the distal end. In other embodiments, the pressure-sensing catheter disclosed herein is configured as a conventional over-the-wire catheter. The pressure-sensing catheters disclosed herein enable the user to obtain pressure measurements using an existing guidewire (e.g., a conventional 0.014 inch guidewire) that can remain fairly stationary through the pressure measurement procedure. Thus, the pressure-sensing catheters disclosed herein enable the user to obtain physiologic information about an intravascular lesion upon pullback of the catheter without losing the original position of the guidewire.

It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description.

### BRIEF DESCRIPTION OF THE DRAWINGS

The accompanying drawings illustrate embodiments of the devices and methods disclosed herein and together with the description, serve to explain the principles of the present disclosure.
FIG. 1 is a diagrammatic partial perspective view of a sensing system according to one embodiment of the disclosure.
FIG. 2 is a diagrammatic partial cross-sectional side view of a micro-catheter sensing system according to an embodiment of the disclosure.
FIG. 3 illustrates a cross-section taken along lines 3-3 of FIG. 2.
FIG. 4 illustrates a cross-section taken along lines 4-4 of FIG. 2.
FIG. 5 is a partial side view of the proximal portion of the micro-catheter of FIG.2.
FIG. 6 is a partial side view of the proximal portion of the micro-catheter of FIG. 5.
FIG. 7 is an end view of the micro-catheter of FIG. 6.
FIG. 8 is a partial cross-sectional view of a sensing micro-catheter according to another aspect of the present disclosure.
FIG. 9 is a diagrammatic partial perspective view of a sensing system according to one embodiment of the disclosure.
FIG. 10A is a diagrammatic partial perspective view of a sensing system according to a second embodiment of the disclosure.
FIG. 10B illustrates a perspective view of an alternative sensing catheter similar to FIG. 10A.
FIG. 11 is a partial perspective view of a further embodiment of a sensing catheter according to another aspect of the present disclosure.
FIG. 12 is partial perspective view of still a further embodiment of a sensing catheter according to another aspect of the present disclosure.

### DETAILED DESCRIPTION

For the purposes of promoting an understanding of the principles of the present disclosure, reference will now be made to the embodiments illustrated in the drawings, and specific language will be used to describe the same. It will nevertheless be understood that no limitation of the scope of the disclosure is intended. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. In addition, dimensions provided herein are for specific examples and it is contemplated that different sizes, dimensions, and/or ratios may be utilized to implement the concepts of the present disclosure. For the sake of brevity, however, the numerous iterations of these combinations will not be described separately. For simplicity, in some instances the same reference numbers are used throughout the drawings to refer to the same or like parts.

The present disclosure relates generally to a device, systems, and methods of using a pressure-sensing catheter for the assessment of intravascular or intravenous pressure, including, by way of non-limiting example, the calculation of a FFR value. In some instances, embodiments of the present disclosure are configured to measure the pressure proximal to and distal to a stenotic lesion within a blood vessel. The pressure-sensing catheters disclosed herein enable the user to obtain pressure measurements using an existing guidewire (e.g., a conventional 0.014 inch guidewire) that can remain fairly stationary through the pressure measurement procedure. Thus, the pressure-sensing catheters disclosed herein enable the user to obtain physiologic information about an intravascular lesion upon pullback of the catheter without losing the original position of the guidewire. In addition, in one aspect, the sensing micro-catheter has an outer diameter of 0.035 inches or less such that conventional peripheral treatment devices may utilize the micro-catheter as a guiding member for positioning the treating device, such as a balloon, in the proper location. Still further, the treatment device may be deployed and withdrawn without moving the micro-catheter sensor such that the pressure can be sensed after treatment to determine effectiveness.

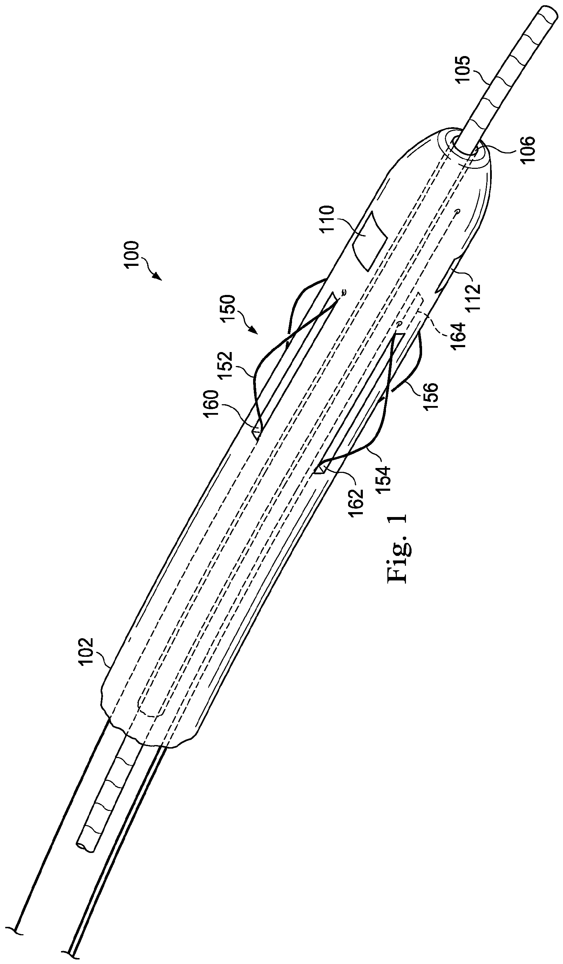

FIG. 1 illustrates a medical system 100 that is configured to measure pressure within a tubular structure V (e.g., a blood vessel) according to one embodiment of the present disclosure. In some embodiments, the medical system 100 is configured to calculate FFR based on the obtained pressure measurements. The system 100 includes a pressure-sensing micro-catheter 102 having two sensors 110 and 112 embedded in the distal portion thereof. FIG. 1 illustrates a pressure sensor 110 embedded in the catheter wall 102. Both for this embodiment and the other embodiments disclosed herein, the pressure sensor 110 comprises any type of pressure sensor that is sufficiently stress resistant to maintain functionality while embedded within the catheter wall. For example, the pressure sensor 110 may comprise a capacitive sensor, a piezoresistive pressure transducer, a fiber optic pressure sensor such as disclosed in U.S. Patent Nos. 8298156 and 8485985 and Application Nos. 2103/0303914 and 2013/0131523, a sensor with a silicon backbone, or any other type of pressure sensor having the requisite durability and stress resistance. In some instances, the sensor 110 includes an array or plurality of sensor elements (e.g., a capacitive pressure sensor array). In the pictured embodiment, the sensor 110 includes a sensor diaphragm assembly. In some embodiments, the sensor diaphragm assembly includes a body having a recess covered by a flexible diaphragm configured to measure fluid pressure. The diaphragm may flex in response to variations in pressure around the diaphragm, thereby reflecting variations in blood pressure, for example. The sensor 110 can then measure and transmit the variations in pressure imparted on the diaphragm assembly. Still further, although the illustrated catheter is described in terms of a pressure sensor, it is contemplated that the type of sensing element disposed on the catheter is not a limitation with respect to all of the teachings of the present disclosure. More specifically, it is contemplated that while one sensor on the catheter may be a pressure sensor, an additional one or more imaging or flow sensors can be incorporated as the sensors of the present disclosure. For example, the sensors 110 and 112 could be ultrasonic transducers that can image the surrounding vessel such as by intravascular ultrasound (IVUS) and/or detect fluid flow in the vessel.

In the illustrated embodiment, the micro-catheter is formed with sensors 110 and 112 utilizing known techniques such as those found U.S. Patent Nos. 6,030,371 and 7,776,3 80. The catheter formation techniques can be utilized to form the channels, passages and electrical conductor members discussed below.

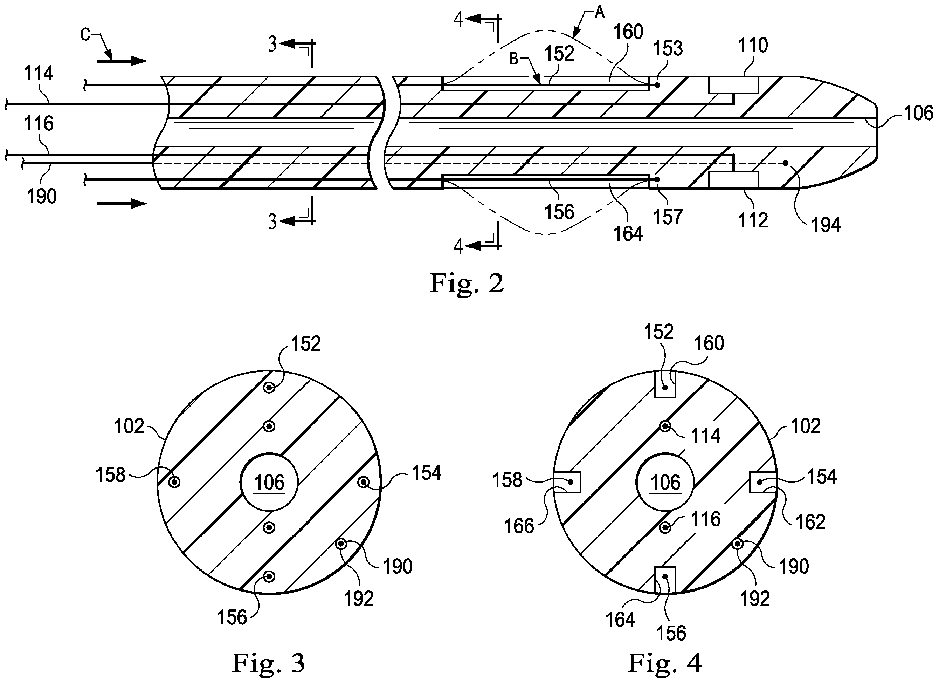

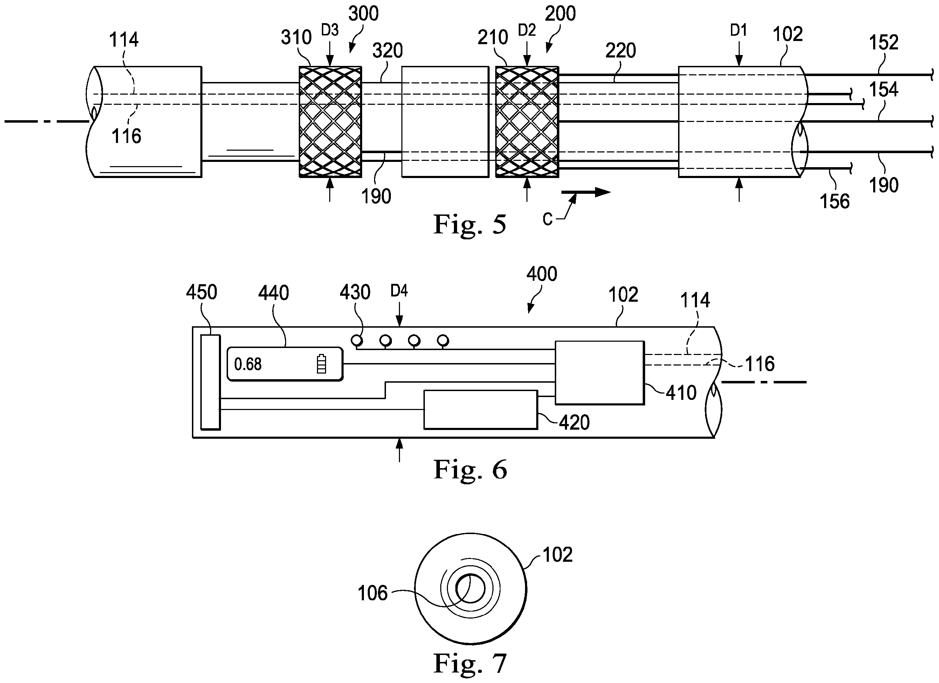

Sensing micro-catheter 102 includes a centering assembly 150 that is configured to extend outwardly from the outer walls of the catheter to engage the surrounding vessel walls and move the sensors away from the vessel walls and into the primary flow of the vessel for more accurate sensor readings. With reference to FIGS 1-4, the centering assembly includes four positioning wires 152, 154, 156 and 158 positioned uniformly around the circumference of the catheter 102. Each of the wires 152, 154, 156 and 158 extend through elongated channels 160, 162, 164 and 166 defined in the outer surface of the catheter body. The positioning wires are anchored in the distal end of the channels such as illustrated at locations 153 and 157 in FIG. 2. The positioning wires extend through and are slidable within tubular lumens (see FIG. 3) of the catheter 102. The positioning wires are joined to circular knob 210 (see FIG. 5) that slides linearly along the catheter body 102 within annular recess 220 to form a steering actuator 200. When knob 210 is moved in the direction of arrow C, the positioning wires are moved in the direction of arrow C until they deform outwardly adjacent the channels in the distal end of the catheter body in a centering configuration A as shown in FIGS. 1 and 2. When the knob is moved in the opposite direction, the positioning wires are withdrawn into the channels into an insertion configuration B as shown in FIG. 2.

In another aspect, the sensing catheter includes a steering mechanism 300 to permit the user to deflect the tip of the catheter to permit the catheter to be steered into the appropriate vessels or around obstructions. The steering mechanism includes a pull wire 190 anchored in the distal tip of the catheter body 102 at location 194 (FIG. 2). The pull wire 190 slidably extends within the catheter body 102 through a small lumen 192 to the proximal end of the catheter where the pull wire is joined to knob 310. Knob 310 is positioned in an annular recess 320 formed on the outside of the catheter body and can slide longitudinal along the catheter body. As the knob is moved forward and backward along the catheter body, the pull wire will act to deflect the distal tip of the catheter.

Sensors 110 and 112 are joined, via electrical conductors 114 and 116, to the proximal processing section 400. Processing section includes an application specific integrated circuit 410 configured to energize the sensors, receive sensor data, process the sensor data and provide outputs to the user through display 440 or through LED's 430. A battery 420 is also disposed within the catheter body 102 to power the ASIC, sensors, display and LED's. Either the display or the LED's can be used to provide battery charge information to the user. An RF coil or antenna 450 may be provided as means for external communication with other devices either to provide sensor output or receive control instruction, or both. Additionally, in some embodiments, the coil may be used for inductive coupling to charge battery 420 or otherwise power the sensors.

With reference to FIGS. 5, 6 and 7, the micro-catheter 102 has a generally uniform diameter D1 along its entire length. More specifically, the diameter D2 of the centering assembly actuator 210 is the same as D1. Likewise the diameter D3 of the steering actuator 310 is the same as D1 and the diameter of the proximal processing assembly D4 is the same as D1. Also, in the illustrated embodiment, the guidewire lumen 106 extends from the distal end to the proximal end. In one embodiment, the guidewire lumen 106 is sized to receive a guidewire 105 having an outer diameter of 0.356 mm (0.014 inch) and the maximum outer diameter of the micro-catheter 102 is no greater than 0.889 mm (0.035 inch) from the distal end to the proximal end.

In use, the guidewire 105 and micro-catheter 106 can be used to traverse a variety of obstacles within a patient. In one aspect, the micro-catheter 106 distal end is significantly more flexible than the guidewire 105. In one use to traverse a tortuous vessel or large curve, the guidewire is retracted about 10 cm within the micro-catheter and the steering assembly is actuated to curve the end of the micro-catheter. The catheter is then advanced along the curve into the desired position. Once in the desired position, the guidewire is advanced through the micro-catheter to stiffen the assembly. This process can be repeated to advance the assembly in the desired direction. In still a further aspect, the guide wire can have a stiff distal portion that can assist the assembly is passing through occlusions that would otherwise deflect the tip of the flexible micro-catheter. Once in the desired location, the sensors can be used to sense vessel characteristics such as pressure and flow of fluids within the vessel, or image the interior of the vessel. A treating device can be advanced over the micro-catheter, deployed to treat the vessel and then withdrawn. The sensors within the micro-catheter can again be utilized to sense the vessel characteristics to determine is the treatment was successful.

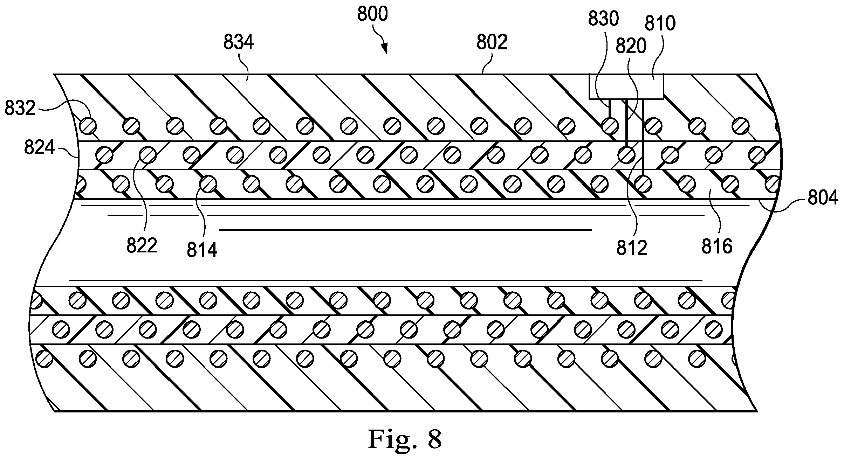

With reference now to FIG. 8, an alternative form of a sensing micro-catheter is illustrated. The micro-catheter 800 includes a sensor 810 and a series of reinforcing fiber layers surrounding an internal guidewire lumen 804. The sensor is electrically connected to the conductive reinforcing filaments 814 via conductor 812 and the filaments 814 are encapsulated by an insulating polymer. Similarly, conductor 820 connects the sensor to conductive filaments 822 which are encapsulated by a non-conductive polymer 824. Finally, conductor 830 connects sensor 810 to conductive filaments 832. The exterior of the catheter body is completed by coating with a non-conductive polymer layer 834. The resulting structure provides a fiber reinforced micro-catheter that has three electrically conductive bands extending the length of the catheter.

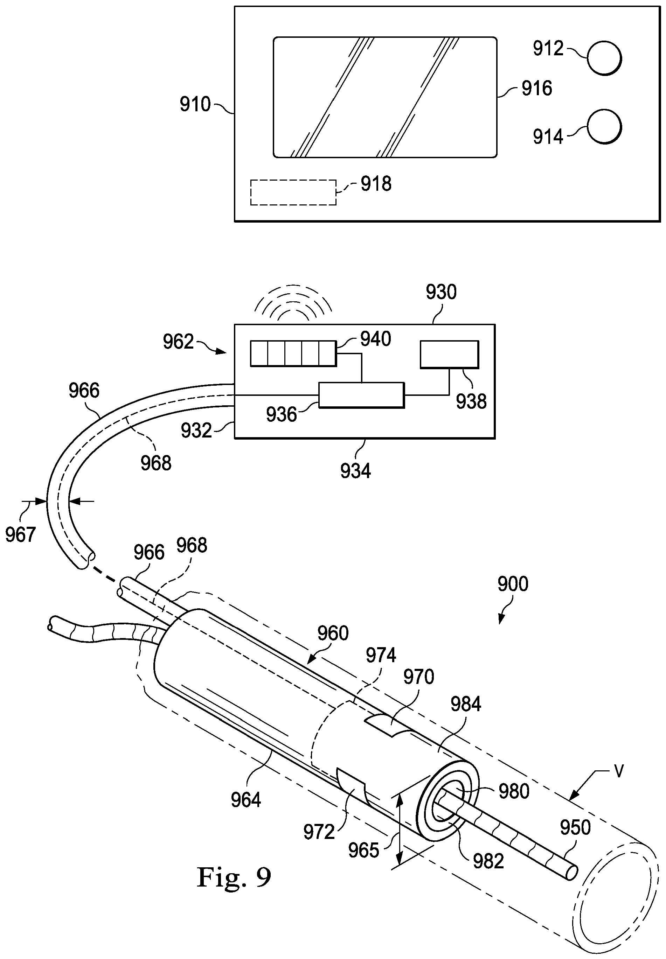

Referring now to FIGS. 9-12, the disclosed embodiments relate generally to a device, systems, and methods of using a pressure-sensing catheter for the assessment of intravascular pressure, including, by way of non-limiting example, the calculation of a FFR value. In some instances, embodiments of the present disclosure are configured to measure the pressure proximal to and distal to a stenotic lesion within a blood vessel. Embodiments of the present disclosure include a pressure sensor embedded in the wall of the catheter or have a movable sleeve capable of smoothing the outer diameter of the sensing catheter. In some embodiments, the pressure-sensing catheter disclosed herein is configured as a monorail or rapid exchange catheter where the guidewire exits the catheter body adjacent the distal end. In other embodiments, the pressure-sensing catheter disclosed herein is configured as a conventional over-the-wire catheter. The pressure-sensing catheters disclosed herein enable the user to obtain pressure measurements using an existing guidewire (e.g., a conventional 0.014 inch guidewire) that can remain fairly stationary through the pressure measurement procedure. Thus, the pressure-sensing catheters disclosed herein enable the user to obtain physiologic information about an intravascular lesion upon pullback of the catheter without losing the original position of the guidewire.

FIG. 9 illustrates a medical system 900 that is configured to measure pressure within a tubular structure V (e.g., a blood vessel) according to one embodiment of the present disclosure. In some embodiments, the medical system 900 is configured to calculate FFR based on the obtained pressure measurements. The system 900 includes a pressure-sensing catheter 960 interconnected with a processing and communication system 930 that is communicatively coupled to a user interface 910.

FIG. 9 illustrates a pressure sensor 970 embedded in the catheter wall 984. Both for this embodiment and the other embodiments disclosed herein, the pressure sensor 970 comprises any type of pressure sensor that is sufficiently stress resistant to maintain functionality while embedded within the catheter wall 984. For example, the pressure sensor 970 may comprise a capacitive sensor, a piezoresistive pressure transducer, a fiber optic pressure sensor such as disclosed in U.S. Patent Nos. 8298156 and 8485985 and Application Nos. 2013/0303914 and 2013/0131523, a sensor with a silicon backbone, or any other type of pressure sensor having the requisite durability and stress resistance. In some instances, the sensor 970 includes an array or plurality of sensor elements (e.g., a capacitive pressure sensor array). In the pictured embodiment, the sensor 970 includes a sensor diaphragm assembly. In some embodiments, the sensor diaphragm assembly includes a body having a recess covered by a flexible diaphragm configured to measure fluid pressure. The diaphragm may flex in response to variations in pressure around the diaphragm, thereby reflecting variations in blood pressure, for example. The sensor 970 can then measure and transmit the variations in pressure imparted on the diaphragm assembly. Still further, although the illustrated catheter is described in terms of a pressure sensor, it is contemplated that the type of sensing element disposed on the catheter is not a limitation with respect to all of the teachings of the present disclosure. More specifically, it is contemplated that while one sensor on the catheter may be a pressure sensor, an additional one or more imaging or flow sensors can be incorporated as the sensors of the present disclosure. For example, the sensors 970 and 972 could be ultrasonic transducers that can image the surrounding vessel such as by intravascular ultrasound (IVUS) and/or detect fluid flow in the vessel.

In the pictured embodiment, the sensor 970 is positioned within a sensor recess defined within the catheter wall. In some embodiments, the sensor 970 is in intimate contact with the wall. The sensor may be coupled to the catheter wall using any of a variety of known connection methods, including by way of non-limiting example, welding, biologically-compatible adhesive, and/or mechanical fasteners. For example, in one embodiment, the sensor is adhesively bonded to the sensor recess using Loctite 3311 or any other biologically compatible adhesive. In some embodiments, the sensors may be integrally formed with the catheter wall. In some embodiments, the sensor recess may be radiopaque.

In one aspect, the sensing catheter 960 is coupled to the processing and communication system 930 such that elongated catheter extension body 966 engages housing 934 while conductors 968 are electrically coupled to application specific integrated circuit (ASIC) 936. Battery 938 powers ASIC 936 and transmitter 940 that communicates wirelessly with user interface 910 in a standard format such as WiFi or Bluetooth. ASIC 936 can provide energizing signals to the sensors 970 and 972 along conductors 968. In one aspect, sensors 970 and 972 are resistive pressure sensors and ASIC 936 receives analog signals from the sensors, processes the signals and provides digital signals to transmitter 940. In one aspect, the proximal end 962 of the catheter 960 is integrally formed with the processing and communication system 930 such that end wall 932 extends directly from catheter body 966 into an enlarged housing area 934 containing the power, processing and communication features of the system. It will be appreciated, that the catheter, including the communication and processing system 930 can be have a unitary, water tight outer surface surrounding the components and that the entire system can be made as a single use disposable item.

User interface 910 includes a wireless communication receiver 918 configured to receive signals from the transmitter 940. The user interface includes processing components (not shown) that can control the display 916 and receive user inputs from buttons 912 and 914. In one form, the raw data provided by the catheter system is displayed directly on display 916. In another form, the user interface 910 is controlled by a user through inputs 912 and 914 to collect sufficient data to calculate a fractional flow reserve (FFR) for a vessel and display that information to the user.

The catheter 960 includes an intermediate portion 966 comprising an elongate, flexible, tubular body extending from the proximal portion 962 to the distal sensing portion 964. The body 966 comprises a catheter wall that defines an internal lumen configured to receive conductors 968. In the illustrated embodiment, intermediate portion 966 has a smaller diameter 967 than the diameter 965 of the distal portion 964. The distal portion 964 includes two embedded sensors 970 and 972. Although two sensors are shown for the purposes of illustration, in some applications only a single sensor is needed, while in other applications a plurality of sensors may be needed. The exterior of sensors 970 and 972 is not greater than the diameter 965 of the distal portion 964 and is generally flush with the outer surface.

The distal portion 964 is constructed by first providing an inner catheter portion 982 defining a guidewire lumen 980 configured to receive a guidewire 150. Sensors 970 and 972 are mounted on the exterior of the inner catheter 982 and electrically connected to the conductors 968. Outer catheter portion 984 is then positioned to surround the inner catheter 982. Outer catheter includes a pair of openings sized to receive the sensors 970 and 972. In one form, the outer catheter 984 is positioned with the openings adjacent the sensors and then heat shrunk to match the outer diameter of the inner catheter 982. In a preferred form, the outer catheter 984 has a material thickness after heat shrinking that is at least as thick as the sensors 970 and 972 extend outwardly from the surface of the inner catheter 982. As a result, as the sensing catheter is advanced along the guidewire within a patient, the sensors will be protected and will not create a protrusion that may catch on patient anatomy.

In general, the catheter 960 is sized and shaped for use within an internal structure of a patient, including but not limited to a patient's arteries, veins, heart chambers, neurovascular structures, gastrointestinal system, pulmonary system, and/or other areas where internal access of patient anatomy is desirable. In the pictured embodiment, the catheter 960 is shaped and sized for intravascular placement.

In particular, the catheter 960 is shaped and configured for insertion into a lumen of a blood vessel V such that a longitudinal axis of the catheter aligns with a longitudinal axis of the vessel at any given position within the vessel lumen. In that regard, the straight configuration illustrated in FIG. 9 is for exemplary purposes only and in no way limits the manner in which the catheter may curve in other instances. Generally, the elongate body may be configured to take on any desired arcuate profile when in the curved configuration. The catheter is formed of a flexible material such as, by way of non-limiting example, plastics, high density polyethylene, polytetrafluoroethylene (PTFE), Nylon, block copolymers of polyamide and polyether (e.g., PEBAX), thermoplastic, polyimide, silicone, elastomers, metals, shape memory alloys, polyolefin, polyether-ester copolymer, polyurethane, polyvinyl chloride, combinations thereof, or any other suitable material for the manufacture of flexible, elongate catheters.

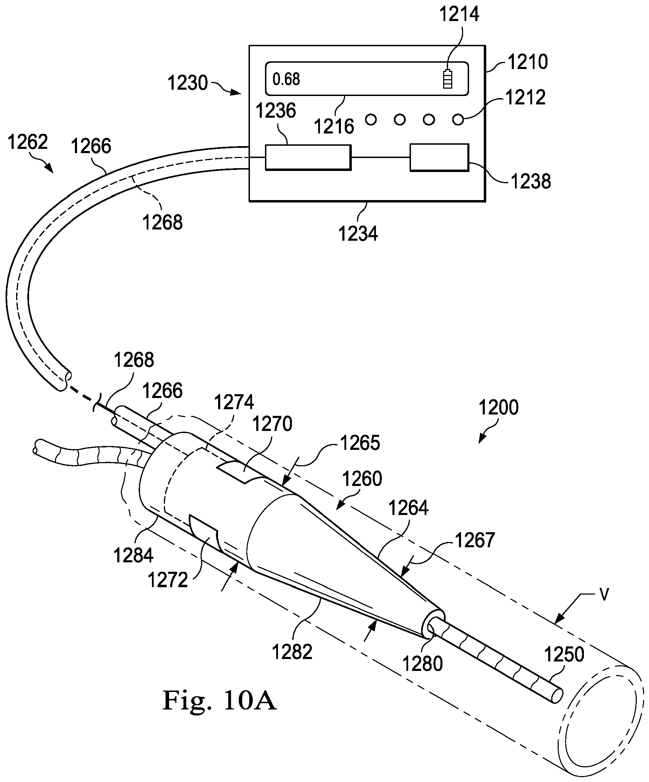

Referring now to FIG. 10A, there is shown still a further embodiment of a sensing catheter according to the present invention. FIG. 10A illustrates a medical system 1200 that is configured to measure pressure within a tubular structure V (e.g., a blood vessel) according to one embodiment of the present disclosure. In some embodiments, the medical system 1200 is configured to calculate FFR based on the obtained pressure measurements. The system 1200 includes a pressure-sensing catheter 1260 interconnected with a processing and communication system 1230. In one aspect, the sensing catheter 1260 is coupled to the processing and communication user interface 1230 such that elongated catheter extension body 1266 engages housing 1234 while conductors 1268 are electrically coupled to application specific integrated circuit (ASIC) 1236 disposed within housing 1234. Battery 1238 powers ASIC 936 and display 1216. In one form, the housing 1234 can also contain a transmitter that communicates wirelessly with a further user interface or other system component in a standard format such as WiFi or Bluetooth. ASIC 936 can provide energizing signals to the sensors 1270 and 1272 along conductors 1268. In one aspect, sensors 1270 and 1272 are resistive pressure sensors and ASIC 1236 receives analog signals from the sensors, processes the signals and provides digital signals the display 1216. In one aspect, the proximal portion 1262 of the catheter 1260 is integrally formed with the processing and communication system user interface 1230 such that housing 1234 extends directly from catheter body 1266 as an enlarged housing area containing the power, processing and communication features of the system. It will be appreciated, that the catheter, including the communication and processing system user interface 1230 can be have a unitary, water tight outer surface surrounding the components and that the entire system can be made as a single use disposable item.

The user interface 1230 includes an ASIC component 1236 that energize on or more sensors 1270 and 1272, receive signals from the sensors, process the sensor data and output results to the display 1216 (and transmit wirelessly if desired). In one form, the raw data provided by the catheter system is displayed directly on display 1216. In another form, the user interface 1230 is controlled by a user through inputs 912 to collect sufficient data to calculate a fractional flow reserve (FFR) for a vessel and display that information to the user. In one example, when positioning the catheter at a first vessel location, a user input 1212 is depressed to take sensor data for a reference pressure. Then at a more distal vessel location, a further button 1212 is depressed to take distal pressure. Using the user interface, the processor 1236 is then utilized to determine the FFR for the distal vessel location. The results of the FFR are then displayed to the user on the display 1216. In the illustrated embodiment, the entire system is powered by a battery 1238. In one aspect, the user interface 1230 can include an induction coil to permit recharging of the battery.

The catheter 1260 includes an intermediate portion 1266 comprising an elongate, flexible, tubular body extending from the proximal portion 1262 to the distal sensing portion 1264. The body 1266 comprises a catheter wall that defines an internal lumen configured to receive conductors 1268. The distal portion 1264 includes two embedded sensors 1270 and 1272. Although two sensors are shown for the purposes of illustration, in some applications only a single sensor is needed, while in other applications a plurality of sensors may be needed. The exterior of sensors 1270 and 1272 is not greater than the diameter 1265 of the distal portion 964 and is generally flush with the outer surface. The distal section of the distal portion 1264 defines outer tapered surface 1282 that transitions from a cylindrical section 1284 containing the sensors to the distal tip having the guidewire lumen 1280 configured to receive the guidewire 1250. The tapered outer surface has an outer diameter 1267 that is less than the diameter 1265.

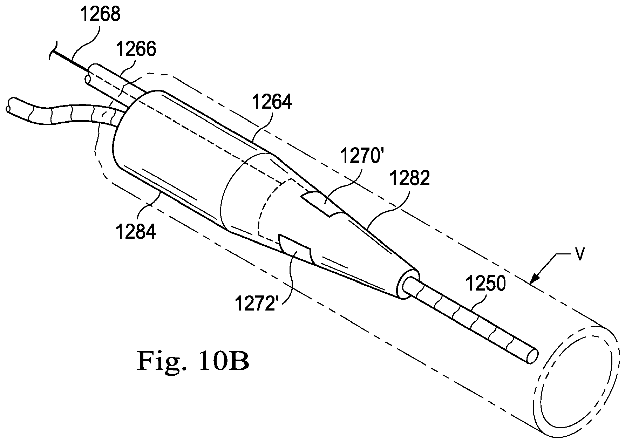

Referring now to FIG. 10B, the illustrated sensing catheter has the same features as those shown in FIG. 10A, with the exception that sensors 1270' and 1272' are positioned along the tapered surface 1282. As illustrated, the sensors are embedded into the catheter such that the sensors do not protrude outwardly from the tapered surface.

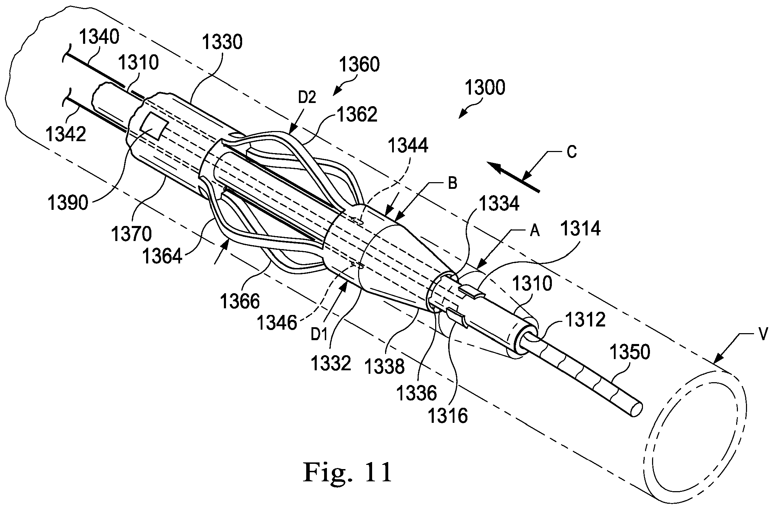

Referring now to FIG. 11, there is shown still a further embodiment of a sensing catheter according to another aspect of the present invention. The sensing catheter 1300 includes an inner catheter 1310 defining an inner lumen 1312 configured to receive a guidewire 1350. A pair of sensors 1314 and 1316 are mounted on the outside of catheter 1310 and extend outwardly therefrom. The sensors are electrically connected to ASIC 1390 which can process analog signals from the sensor and transmit corresponding data signals to the proximal end of the catheter. An outer catheter 1330 is mounted about inner catheter 1310. Proximal portion 1370 is fixed in relation to inner catheter 1310 while distal portion 1332 is moveably mounted on the inner catheter. A centering assembly 1360 is defined by the outer catheter between the proximal portion 1370 and the distal portion 1332. In the illustrated embodiment, the centering assembly 1360 includes a plurality of elongated deformable legs 1362, 1364, and 1366 defined by cutting elongated apertures in the outer catheter 1330. A pair of pull wires 1340 and 1342 extend through lumens within the outer catheter 1330, across the centering assembly and are anchored in the distal portion 1332 at anchor points 1344 and 1346, respectively. The distal portion 1332 includes a pair of groove 1334 and 1336 sized to slidably receive the sensors 1314 and 1316, respectively, protruding from the inner catheter 1310. The entrance to each groove 1334 and 1336 begins in the tapered end 1338.

In operation, the catheter assembly is initially in an insertion configuration with the distal portion 1332 extended to the position A with the centering assembly in a collapsed configuration having an outer diameter substantially matching diameter D1. In one aspect, the material of outer catheter 1330 has sufficient resiliency such that when no force is applied to the pull wires 1340 and 1342, the centering assembly is biased to return to the collapsed configuration. The center is advanced over the guidewire 1350 to position the sensors in the desired location. When force is applied to the pull wires 1340 and 1342 in the direction of arrow C, the distal portion 1332 slides longitudinally along the inner catheter 1310 to the position B to expose the sensors 1314 and 1316. Since the distal portion 1370 of the outer catheter is fixed in relation to the inner catheter the elongated legs 1362, 1364, and 1366 (along with a forth leg not shown), deform outwardly to the centering configuration illustrated in FIG. 11. In the centering configuration the legs have an outer diameter of D2 that is substantially larger than diameter D1. Thus, as shown, the centering assembly centers the sensors in the middle of the vessel V. The deformable legs are sized to limit their impact on the blood flow in the vessel. In this way, the sensors are positioned in the ideal location within the vessel to obtain the best possible reading of the fluid, including such characteristics as pressure and flow.

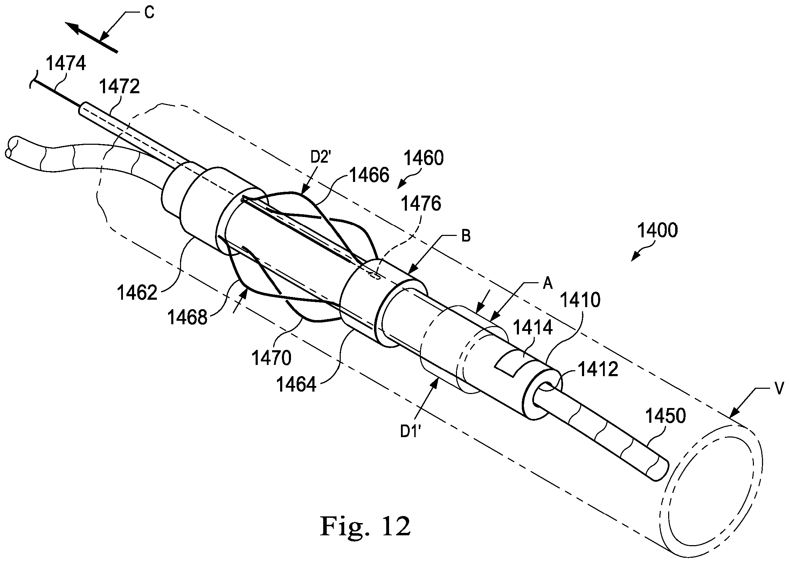

Referring now to FIG. 12, there is shown still a further embodiment of a sensing catheter 1400 having a sensor 1414 disposed on a distal catheter body 1410 defining guidewire lumen 1412 that receives guidewire 1450. The catheter 1400 includes a centering assembly 1460 comprising a distal ring 1464 slidably mounted on the distal catheter body 1410 and a proximal ring 1462 fixed to the catheter body 1410. A plurality of wires 1466, 1468 and 1470 (other wires on the back side are not shown) interconnect the distal ring and the proximal ring. A pull wire 1474 extends through proximal catheter body 1472 into the distal catheter body 1410 and is connected to distal ring 1464 at anchor point 1476. Tension applied on pull wire 1474 in the direction of arrow C moves distal ring 1464 toward proximal ring 1462, thereby deforming wires 1466, 1468, and 1470 outwardly to the centering configuration shown in FIG. 12. In the centering configuration with distal ring 1464 positioned at position B, the assembly has an outer diameter of D2' that is much larger than the diameter D1' of the assembly in the insertion configuration. The wires 1466, 1468 and 1470 are resilient and bias the assembly into the collapsed insertion configuration with distal ring positioned at position A. In the illustrated embodiment, the centering assembly is mounted on the exterior of the catheter 1410, however, it is contemplated that in an alternative embodiment, an annular groove may extend around the catheter 1410 such that in the collapsed position, the centering assembly has a maximum outer diameter that is less than or the same as the diameter of the catheter 1410.

With reference to the above described embodiments, the guidewire lumen includes an internal diameter that is sized and shaped to accommodate the passage of a standard guidewire. The internal diameter may range from 0.356 to 10.16 mm (0.014 to 0.40 inch) In one embodiment, the internal diameter is 0.406 mm (0.016 inch) to slidingly accommodate a 0.356 mm (0.014 inch) diameter guidewire. In one embodiment, the internal diameter is 0.61 mm (0.024 inch) In one embodiment, the internal diameter is 0.457 mm (0.018 inch) In another embodiment, the internal diameter is 0.965 mm (0.038 inch). to accommodate a 0.889 mm (0.035 inch) diameter guidewire. The catheter includes an outer diameter that is sized and shaped to traverse bodily passageways. In the pictured embodiment, the outer diameter is sized to allow passage of the catheter through vascular passageways. In some instances, as mentioned above, the body has an external diameter ranging 0.356 to 1.27 mm (0.014 to 0.050 inch)

In one embodiment, the outer diameter is 0.61 mm (0.024 inch) with an internal diameter of about 0.406 mm (0.016 inch). In one embodiment, the outer diameter is 0.457 mm (0.018 inch). In another embodiment, the outer diameter is 0.889 mm (0.035 inch).

Persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. For example, the pressure-sensing catheters disclosed herein may be utilized anywhere with a patient's body, including both arterial and venous vessels, having an indication for pressure measurement.

## Claims

1. An apparatus for intravascular pressure measurement, comprising:
an elongate body including a proximal portion (962) and a distal portion (964), the body defining a lumen (106) extending from a proximal end to a distal end of the body, the lumen sized and shaped to allow the passage of a guidewire (105) therethrough, the body including an annular wall (102) extending from the lumen to an outer surface of the body;
a first pressure sensor disposed within the wall (102) of the distal portion of the body; and
a centering assembly (150) disposed adjacent the pressure sensor, the centering assembly having a first collapsed position and a second expanded position adapted to engage a vessel wall to center the pressure sensor within the vessel, **characterized in that**:
the centering assembly comprises positioning wires (152, 154, 156, 158) configured to engage the vessel wall, the positioning wires extending through and anchored in distal ends of elongated channels (160, 162, 164, 166) defined in the outer surface of the elongate body and further extending through and slidable within tubular lumens of the annular wall (102).

2. The apparatus of claim 1, wherein an exterior surface of the sensor and the outer surface of the body are substantially aligned to form a smooth outer surface.

3. The apparatus of claim 1, wherein the outer surface of the body has a proximal diameter and at least one distal taper have a reduced diameter adjacent the distal portion.

4. The apparatus of claim 1, wherein the centering assembly includes at least three outwardly extending legs.

5. The apparatus of claim 1, wherein the positioning wires are joined to a circular knob (210) linearly slidable within annular recesses (220) along a portion of the elongate body proximal to the tubular lumens.

6. The apparatus of claim 1, wherein the centering assembly is recessed within the outer surface of the body when in the collapsed position.

7. The apparatus of claim 3, wherein the pressure sensor is disposed within the distal taper.

8. The apparatus of claim 1, wherein the outer surface of the body has a diameter of 0.889 mm (0.035 inches) or less.

9. The apparatus of claim 7, wherein the lumen has an internal diameter of at least 0.356 mm (0.014 inches) and the sensor is disposed between the lumen and the outer surface.

10. The apparatus of claim 1, further including sensor cover mounted on the outer surface of the body, the sensor cover movable from a distal sensor covering position to a proximal sensor exposed position spaced proximally from the sensor.

11. The apparatus of claim 10, wherein the sensor cover includes a tapered distal end.

12. The apparatus of claim 10, wherein the sensor cover includes a recess to receive at least a portion of the sensor protruding outwardly from the outer surface of the body.

13. The apparatus of claim 1, wherein the elongated body is at least 50 centimeters in length.

14. The apparatus of claim 1, further including a plurality of the reinforcing wires extending along the length of the elongate body, wherein a least one wire is electrically connected to the pressure sensor.

15. The apparatus of claim 1, further including a plurality of pressure sensors disposed within the wall of the distal portion of the body.

## Patentansprüche

1. Vorrichtung zur Messung eines intravaskulären Drucks, umfassend:
einen länglichen Körper, der einen proximalen Abschnitt (962) und einen distalen Abschnitt (964) umfasst, wobei der Körper ein Lumen (106) definiert, das sich von einem proximalen Ende zu einem distalen Ende des Körpers erstreckt, wobei das Lumen so bemessen und geformt ist, dass es ermöglicht, dass ein Führungsdraht (105) dort hindurch verläuft, wobei der Körper eine ringförmige Wand (102) aufweist, die sich von dem Lumen zu einer Außenfläche des Körpers erstreckt;
einen ersten Drucksensor, der innerhalb der Wand (102) des distalen Abschnitts des Körpers angeordnet ist; und
eine Zentrierungsbaugruppe (150), die angrenzend an den Drucksensor angeordnet ist, wobei die Zentrierungsbaugruppe eine erste kollabierte Position und eine zweite aufgeweitete Position aufweist, die dafür eingerichtet ist, an einer Gefäßwand anzugreifen, um den Drucksensor innerhalb des Gefäßes zu zentrieren, **dadurch gekennzeichnet, dass**:
die Zentrierungsbaugruppe Positionierungsdrähte (152, 154, 156, 158) umfasst, die dafür ausgelegt sind, an der Gefäßwand anzugreifen, wobei sich die Positionierungsdrähte durch distale Enden von länglichen Kanälen (160, 162, 164, 166), die in der Außenfläche des länglichen Körpers definiert sind, erstrecken und darin verankert sind und sich weiter durch röhrenförmige Lumen der ringförmigen Wand (102) erstrecken, in denen sie verschiebbar sind.

2. Vorrichtung nach Anspruch 1, wobei eine Außenfläche des Sensors und die Außenfläche des Körpers im Wesentlichen so aneinander ausgerichtet sind, dass sie eine glatte Außenfläche bilden.

3. Vorrichtung nach Anspruch 1, wobei die Außenfläche des Körpers einen proximalen Durchmesser aufweist und mindestens eine distale Verjüngung angrenzend an den distalen Abschnitt einen verringerten Durchmesser aufweist.

4. Vorrichtung nach Anspruch 1, wobei die Zentrierungsbaugruppe mindestens drei sich auswärts erstreckende Schenkel aufweist.

5. Vorrichtung nach Anspruch 1, wobei die Positionierungsdrähte an einen kreisförmigen Knauf (210) angefügt sind, der innerhalb ringförmiger Aussparungen (220) entlang eines Abschnitts des länglichen Körpers proximal zu den röhrenförmigen Lumen verschiebbar ist.

6. Vorrichtung nach Anspruch 1, wobei die Zentrierungsbaugruppe in der Außenfläche des Körpers zurückgesetzt ist, wenn sie die kollabierte Position einnimmt.

7. Vorrichtung nach Anspruch 3, wobei der Drucksensor innerhalb der distalen Verjüngung angeordnet ist.

8. Vorrichtung nach Anspruch 1, wobei die Außenfläche des Körpers einen Durchmesser von 0,889 mm (0,035 Inch) oder weniger aufweist.

9. Vorrichtung nach Anspruch 7, wobei das Lumen einen Innendurchmesser von mindestens 0,356 mm (0,014 Inch) aufweist und der Sensor zwischen dem Lumen und der Außenfläche angeordnet ist.

10. Vorrichtung nach Anspruch 1, ferner eine Sensorabdeckung aufweisend, die an der Außenfläche des Körpers montiert ist, wobei die Sensorabdeckung von einer distalen, den Sensor abdeckenden Position zu einer proximalen, den Sensor freilassenden Position, die proximal von dem Sensor beabstandet ist, bewegbar ist.

11. Vorrichtung nach Anspruch 10, wobei die Sensorabdeckung ein sich verjüngendes distales Ende aufweist.

12. Vorrichtung nach Anspruch 10, wobei die Sensorabdeckung eine Aussparung aufweist, um zumindest einen Abschnitt des Sensors aufzunehmen, der von der Außenfläche des Körpers nach außen vorsteht.

13. Vorrichtung nach Anspruch 1, wobei der längliche Körper eine Länge von mindestens 50 Zentimetern aufweist.

14. Vorrichtung nach Anspruch 1, ferner eine Mehrzahl von Verstärkungdrähten aufweisend, die sich entlang der Länge des länglichen Körpers erstrecken, wobei mindestens ein Draht elektrisch mit dem Drucksensor verbunden ist.

15. Vorrichtung nach Anspruch 1, ferner eine Mehrzahl von Drucksensoren aufweisend, die innerhalb der Wand des distalen Abschnitts des Körpers angeordnet sind.

## Revendications

1. Appareil de mesure de la pression intravasculaire, comprenant :
un corps allongé comprenant une partie proximale (962) et une partie distale (964), le corps définissant une lumière (106) s'étendant de l'extrémité proximale à l'extrémité distale du corps, la lumière étant dimensionnée et formée pour permettre le passage d'un fil de guidage (105) à travers celle-ci, le corps comprenant une paroi (102) annulaire s'étendant depuis la lumière vers une surface externe du corps ;
un premier capteur de pression disposé à l'intérieur de la paroi (102) de la partie distale du corps ; et un ensemble de centrage (150) disposé adjacent au capteur de pression, l'ensemble de centrage présentant une première position repliée et une seconde position déployée apte à l'engagement d'une paroi de vaisseau pour centrer le capteur de pression à l'intérieur du vaisseau, **caractérisé en ce que** :
l'ensemble de centrage comprend des fils de positionnement (152, 154, 156, 158) conçus pour venir en prise avec la paroi du vaisseau, les
fils de positionnement s'étendant à travers et étant ancrés dans les extrémités distales de canaux (160, 162, 164, 166) allongés définis dans la surface externe du corps allongé et s'étendant en outre à travers et coulissant à l'intérieur des lumières tubulaires de la paroi (102) annulaire.

2. Appareil selon la revendication 1, dans lequel une surface extérieure du capteur et la surface extérieure du corps sont sensiblement alignées pour former une surface extérieure lisse.

3. Appareil selon la revendication 1, dans lequel la surface extérieure du corps présente un diamètre proximal et au moins un cône distal présente un diamètre réduit adjacent à la partie distale.

4. Appareil selon la revendication 1, dans lequel l'ensemble de centrage comprend au moins trois branches s'étendant vers l'extérieur.

5. Appareil selon la revendication 1, dans lequel les fils de positionnement sont réunis au niveau d'un bouton (210) circulaire coulissant linéairement dans des évidements (220) annulaires le long d'une partie du corps allongé à proximité des lumières tubulaires.

6. Appareil selon la revendication 1, dans lequel l'ensemble de centrage est encastré dans la surface extérieure du corps lorsqu'il est dans la position repliée.

7. Appareil selon la revendication 3, dans lequel le capteur de pression est disposé à l'intérieur du cône distal.

8. Appareil selon la revendication 1, dans lequel la surface extérieure du corps présente un diamètre de 0,889 mm (0,035 pouces) ou inférieur.

9. Appareil selon la revendication 7, dans lequel la lumière présente un diamètre interne d'au moins 0,356 mm (0,014 pouces) et le capteur est disposé entre la lumière et la surface externe.

10. Appareil selon la revendication 1, comprenant en outre un recouvrement de capteur monté sur la surface extérieure du corps, le recouvrement de capteur pouvant être déplacé d'une position de recouvrement de capteur distale à une position exposée de capteur proximale espacée de manière proximale du capteur.

11. Appareil selon la revendication 10, dans lequel le recouvrement de capteur comprend une extrémité distale effilée.

12. Appareil selon la revendication 10, dans lequel le recouvrement de capteur comprend un évidement pour recevoir au moins une partie du capteur faisant saillie vers l'extérieur depuis la surface externe du corps.

13. Appareil selon la revendication 1, dans lequel le corps allongé mesure au moins 50 centimètres de longueur.

14. Appareil selon la revendication 1, comprenant en outre une pluralité de fils de renforcement s'étendant le long de la longueur du corps allongé, dans lequel au moins un fil est électriquement connecté au capteur de pression.

15. Appareil selon la revendication 1, comprenant en outre une pluralité de capteurs de pression disposés à l'intérieur de la paroi de la partie distale du corps.
